(19) Europäisches Patentamt / European Patent Office / Office européen des brevets

(11) **EP 3 138 478 B1**

(12) **EUROPEAN PATENT SPECIFICATION**

(45) Date of publication and mention
of the grant of the patent:
**01.11.2023  Bulletin 2023/44**

(21) Application number: **15306343.3**

(22) Date of filing: **01.09.2015**

(51) International Patent Classification (IPC):
**A61B 5/00** *(2006.01)*      **A61B 5/024** *(2006.01)*

(52) Cooperative Patent Classification (CPC):
**A61B 5/02438; A61B 5/6803; A61B 5/6886;**
**A61B 5/7278;** A61B 2562/0257

(54) **A HEART RATE SENSING WEARABLE DEVICE**

TRAGBARE HERZFREQUENZMESSVORRICHTUNG

DISPOSITIF PORTABLE DE DÉTECTION DE FRÉQUENCE CARDIAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.03.2017  Bulletin 2017/10**

(60) Divisional application:
**23194372.1 / 4 260 802**

(73) Proprietor: **Essilor International
94220 Charenton-le-Pont (FR)**

(72) Inventors:
• **ROUSSEAU, Denis
94227 Charenton-le-Pont Cedex (FR)**
• **PERRIER, Eric
94227 Charenton-le-Pont Cedex (FR)**

(74) Representative: **Cabinet Novitech
188 Grande rue Charles de Gaulle
94130 Nogent-sur-Marne (FR)**

(56) References cited:
WO-A1-2010/140106      WO-A2-2013/049248
US-A1- 2014 200 469      US-A1- 2015 045 650
US-A1- 2015 051 468

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates to a wearable device comprising a heart rate determining component.

BACKGROUND OF THE INVENTION

Sensing heart rate of a user of a wearable device may provide useful information concerning the user, in particular concerning the health condition of the user. Most of the existing heart rate sensing devices requires a contact with the skin of the user. When using a wearable device on an everyday bases it may be complex ensuring permanent contact between the heart rate sensing device and the skin of the user.

The documents US 2015/051468 A1, US2015/045650 A1, WO2010/140106 A1 and US2014/200469 A1 describe known wearable heart rate sensing devices.

Thus there is a need for heart rate sensing module that would not require contact with the user's skin.

One object of the present invention is to provide a wearable device for sensing a heart rate.

SUMMARY OF THE INVENTION

To this end, the invention proposes a device as defined in claim 1.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") or FPGA selectively activated or reconfigured by a computer program or computer executable instructions stored in the computer. Such a computer program or computer executable instructions may be stored in a computer readable storage medium, such as, but is not limited to, any type of disk including floppy disks, optical disks, CD-ROMs, magnetic-optical disks, read-only memories (ROMs), random access memories (RAMs), ferro magnetic RAM, electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

BRIEF DESCRIPTION OF THE DRAWINGS

Embodiments of the invention will now be described, by way of example only, and with reference to the following drawings in which:

- Figure 1 is a schematic representation of a heart rate sensing module according to the invention,
- Figure 2 is a schematic representation of a heart rate determining component according to the invention,
- Figure 3 is a schematic representation of an head mounted device according to an embodiment of the invention;
- Figure 4 represents a networked data-processing device according to the invention; and
- Figure 5 is a schematic representation of a head mounted device according to a further embodiment of the invention.

DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

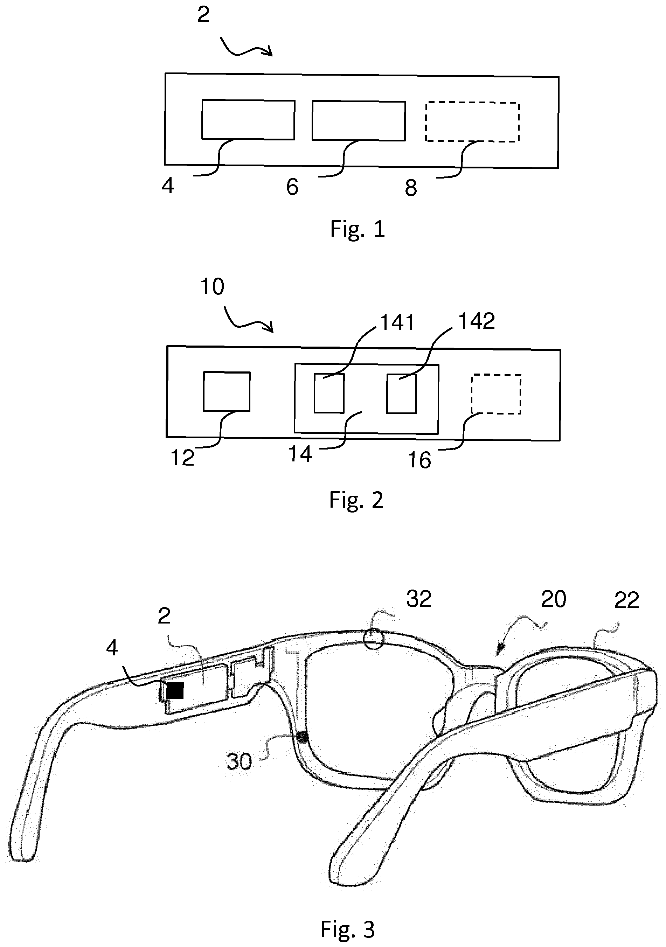

As represented on figure 1, the disclosure relates to a heart rate sensing module 2. The heart rate sensing module is fixed on a wearable device, the wearable device being intended to be worn by a user.

The wearable device may be a head mounted device such as a spectacle frame, as represented on figure 2, or a pair of shoes or a watch.

The heart rate sensing module 2 comprises at least a contactless sensor 4 and a communication entity 6.

The contactless sensor 4 is adapted to sense at least one parameter indicative of the distance between at least a reference point of the skin of the user and a reference point of the module, in particular of the contactless sensor, when the module is fixed to the wearable device when worn by the user in such a manner that the contactless sensor is distant from the skin of the user.

Preferably, when the heart rate sensing module is fixed on the wearable device and said wearable device is worn by the user the distance between the contactless sensor, in particular the at least one reference point of the contactless sensor and the skin of the user, in particular the reference point of the of the skin of the use, is greater than or equal to 0.1 mm, for example greater than or equal to 1 mm and smaller than or equal to 1 cm, for example smaller than or equal to 7 mm.

So as to provide more accurate heart rate sensing, the heart rate sensing module may be configured to that when fixed on the wearable device and said wearable device worn by the user, the contactless sensor faces an artery or vein of the user.

The contactless sensor may be a capacitive sensor. The capacitance measured by the capacitive sensor may be used as an indication of the distance between the sensor and the skin of the user.

Typically, the electrode of the capacitive sensor can be printed on a circuit board fixed onto a wearable device or can be mounted directly into the wearable device or printed with conductive ink directly on the wearable device. The electrode may be flexible and the sensor may be embedded into clothes, for example using conductive wires like sliver nanowires embedded into standard clothes.

When the electrode of the capacitive sensor is approached by a distant object, like a finger or human skin, the capacitance of the circuit changes. The formula to determine capacitance is as follow :

$$C = (\varepsilon 0 * \varepsilon r) * S/e \, ,$$

with

$\varepsilon 0$ is the air dielectric constant,
$\varepsilon r$ is the relative dielectric constant of the material between skin and electrode,
e is the distance between the electrode and the distant object,
S is the surface of the electrodes

Blood when flowing in the vein and arteria induces a vein and arteria diameter variation and induces a small skin variation. Such skin variation can be detected by a capacitance measurement between the sensor and the skin of the user facing the sensor at a distance greater than or equal to 1 mm and smaller than or equal to 1cm.

Standard electronic chips are available to measure the capacitance variation induced by the distance variation between the sensor and the skin due to blood flow.

The contactless sensor may also be a magnetic sensor measuring the variation of magnetic field between two magnetic elements, one on the wearable device and one on the skin of the user, for example a magnet is stuck on the user's skin. In this case the magnetic sensor is on the wearable device and measure the magnetic field variation due to skin movement when blood flow.

The communication entity 6 of the heart rate sensing module 2 is adapted to communicate data indicative of the at least one parameter sensed by the at least one sensor to a heart rate determining component.

The data indicative of the at least one parameter sensed by the at least one sensor may be filtered to improve the accuracy, for example using a specific software and by selecting only the bandwidth of the heartbeat. This could be done using Fourrier transform or other frequency filtering methods.

As illustrated on figure 1, the heart rate sensing module may comprise a memory 8 to store the data prior to sending them to a heart rate determining component.

The heart rate sensing module may further comprise a power source, such as a battery, or may be electrically connected to a power source of a heart rate determining component or a power source comprised in the wearable device.

The disclosure further relates to a heart rate determining component for determining the heart rate of a wearer of a wearable sensing device according to the invention.

As illustrated on figure 2, the heart rate determining component 10 according to the disclosure comprises a communication unit 12 and a heart rate unit 14.

The communication unit 12 is configured to receive data indicative of the at least one parameter sensed by the at least one sensor of a heart rate sensing module according to the disclosure.

The communication unit 12 communicates with the communication entity 6 of a heart rate sensing module of the disclosure.

Such communication may be wireless or both communication units may be linked electrically. Wireless communication can be done through different communication and protocols, like Bluetooth, Zigbee, WiFi or others.

The heart rate unit comprises at least a memory 141 and a processor 142. The memory 141 stores computer executable instructions. In addition to the computer executable instructions, the memory may store data such as at least part of the data received by the communication unit 12 or part of the data determined by the processor when executing the computer executable instructions stored in the memory.

The processor 142 is configured to execute the computer executable instructions stored in the memory 141. The stored computer executable instructions comprise instructions for determining heart rate data indicative of the heart rate of the user based on the received data.

According to an embodiment, the computer executable instructions further comprises instructions for determining heart rate data based on the variation over time of the distance between the at least a reference point of the skin of the user and a reference point of the module when the module is fixed to the wearable device worn by the user.

As illustrated on figure 2, the heart rate determining component 10 may further comprise an information generating unit 16 configured to generate information based on the heart rate data.

The information may comprise the information comprises recommendation data including a change of activity recommendation and/or an alert indicative of the user's heart state and/or an access to a service offer.

The communication unit 12 may be further configured to send heart rate related data to a distant entity, for example a wearable computer device and/or a personal computer device, associated to the user of the heart rate determining component. The heart rate related data may be information generated by the information generating unit 16 or data indicative of the heart rate of the user to be processed by the distant entity.

The heart rate determining component may further comprise a power source, such as a battery, or may be electrically connected to a power source of a heart rate sensing module or a power source comprised in the wearable device.

The invention further relates to a wearable device comprising a heart rate module. The wearable device further comprises a heart rate determining component arranged to receive data from the heart rate module.

Figure 3 illustrates an example of a wearable device, for example a head-mounted device 20, comprising a heart rate sensing module 2. The wearable device further comprises different type of sensors 30, 32. The head mounted device 20 comprises a spectacle frame 12 and the sensors and communication unit are mounted on the spectacle frame 12.

Although the invention is not limited to such type of wearable device, head-mounted device, appear to be particularly advantageous, in particular head mounted devices comprising a spectacle frame.

Indeed, such type of head mounted device is more likely to positioned over a long period of time facing a skin of the wearer, in particular facing the posterior auricular artery or vein.

The sensors 30 and 32 may be configured to sense at least one feature indicative of the relative position of the head mounted device and at least one reference point of the head of the user of the head mounted device.

Advantageously, having an indication of the relative position of the head mounted device and the head of the user can be used to increase the accuracy of the measurements of the heart rate using the heart rate sensing module of the invention. Indeed, the heart rate sensing module senses data indicative of the distance between the sensor and the skin of the user. Thus, having an indication of the relative position of the sensor and the skin over time may be correlated to the data provided by the heart rate sensing module to increase the accuracy.

At least one of the additional sensors may be a time of flight sensor configured to sense the distance between the head mounted device and the user of the head mounted device, for example the user's head. Such sensor may be placed on the frame in such position that when the head mounted device is worn by the user the sensor points to the face of the wearer. The sensor typically uses time of flight (TOF) technology and is arranged to provide data indicative of the distance to the head depending of the position of the head mounted device.

According to an embodiment of the invention, at least one of the additional sensor is a proximity sensor based on infrared reflectance on the user skin so as to sense the proximity between the head mounted device and the user of the head mounted device, for example the user's head.

Such infrared reflectance sensor may advantageously be placed on the nose bridge of the frame of the head mounted device and oriented toward the nose so as to provide data indicative of the distance to the skin of the user, for example of the sellion of the user. Advantageously, when the head mounted device is worn by the user, the nose bridge sensor is very close to the skin of the user, and if the position of the head mounted device changes this distance increases and can be easily detected.

The time of flight and the infrared reflectance sensors may require training to detect distance during the different use of the head mounted device, and detection of the different positions occur after statistical analysis of the data provided by the sensors.

According to a further embodiment of the invention, one of the additional sensors is an accelerometer and/or gyroscope configured to sense the orientation of the head mounted device.

Typically, in a first reference position, the head mounted device is almost horizontal, and in other position the head mounted device may be oriented upward or downward, this inclination can be measured by 3 or 6 axis sensors commonly used in mobile phone. Typically, a 3 axis accelerometer can provide data concerning the horizontality of the head mounted device and a 3 axis gyroscope can provide data concerning rotation of the head mounted device. Also the movements of the head mounted device from one position to another can be detected, and differentiated from head movement to be sure to detect a head mounted device relative movement. In some case only accelerometer can be used, to save power.

The different type of sensors provide features indicative that need to be processed so as to determine information indicative of the relative position of the head mounted device and the user of the head mounted device.

Although not represented, the head mounted device may further comprise a power source, for example a battery and/or other electronics.

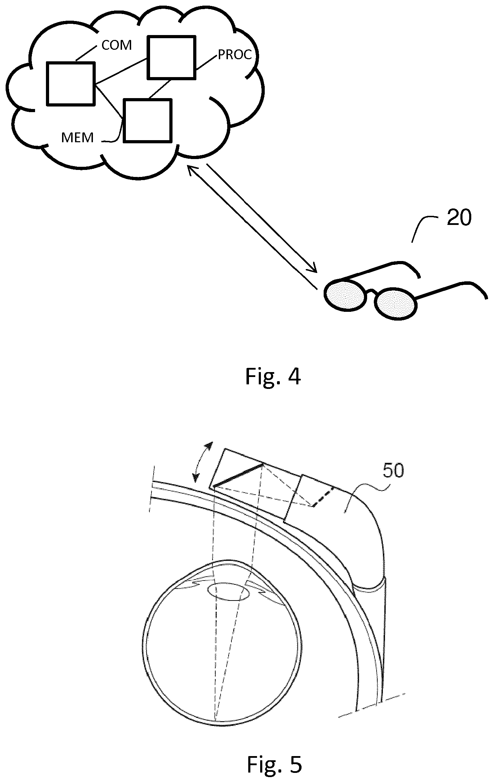

According to an embodiment of the invention, illustrated on figure 4, the head mounted device communicates with a distant entity that comprises a heart rate determining component. Communication can be done through different communication devices and protocols, like Bluetooth, Zigbee, WiFi or others.

For example, the communication unit is configured to communicate with the distance entity either to store the measured features in a memory MEM or to provide information relating to the heart rate of the user.

Typically, the distant entity comprises a heart rate determining component according to the invention.

The distance entity can include different computing objects such as personal digital assistants, audio/video devices, mobile phones, MPEG-1 Audio Layer 3 (MP3) players, personal computers, laptops, tablets, bluetooth headset, watch, wristband, etc...

Each computing object and the head mounted device can communicate with one or more other by way of a communication network, either directly or indirectly. Even though illustrated as a single element in figure 4, network can include other computing objects and computing devices that provide services to the system of figure 4, and/or can represent multiple interconnected networks, which are not shown.

In a network environment in which the communications network/bus can be the Internet, the computing objects can be Web servers, file servers, media servers, etc. with which the client computing objects or devices communicate via any of a number of known protocols, such as the hypertext transfer protocol (HTTP).

In an alternative embodiment, the heart rate determining component may be embodied in the wearable device and electrically connected to the heart rate sensing module.

According to an embodiment of the invention, the data received by the heart rate determining component from the heart rate sensing module may be stored over time and the information is indicative of the evolution over time of the heart rate of the user.

Furthermore the information generating unit may be configured to generate information using statistical analysis of the data received from the head mounted device and stored over time.

Statistics involves the collection, organization, analysis, interpretation, and/or presentation of measured/collected information. With advances in technology, more extensive and complex computing allows massive amounts of data to be collected, stored and/or processed. Further, methods for evaluating the data are numerous.

Statistical analysis can be employed to process and/or evaluate data sensed.

As represented on figure 4, the head mounted device according to the invention may comprise a virtual image display device 50, preferably allowing the wearer to see both the virtual image and the real world through it. The virtual image display device is able to display graphical images, and an electronic driving system (memory + processor) sends to the virtual display image the image to display. For example the heart rate over time may be displayed or an alert may be displayed if a heart or health defect is detected.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. A wearable device comprising a heart rate determining component (10) for determining the heart rate of a wearer of the wearable device and a heart rate sensing module (2) fixed on the wearable device, the wearable device intended to be worn by a user, the module comprising:

   at least one contactless sensor (4) adapted to sense at least one parameter indicative of the distance between at least a reference point of the skin of the user and a reference point of the module when the wearable device is worn by the user in such a manner that the contactless sensor is distant from the skin of the user, at least one communication entity (6) adapted to communicate data indicative of the at least one parameter sensed by the at least one contactless sensor to the heart rate determining component, the heart rate determining component comprising:

   a communication unit (12) configured to receive data indicative of the at least one parameter sensed by the at least one contactless sensor (4) of the heart rate sensing module, and a heart rate unit (14) comprising at least a memory (141) configured to store computer executable instructions; and
a processor (142) for executing the computer executable instructions, wherein the computer executable instructions comprises instructions for determining heart rate data indicative of the heart rate of the user based on the received data, **characterised in that** the wearable device further comprises
one of a time of flight sensor configured to sense the distance between the wearable device and the user of the wearable device and an infrared reflectance based proximity sensor to sense the proximity between the wearable device and the user of the wearable device,
and **in that** the wearable device further comprises
an accelerometer and/or a gyroscope configured to sense the orientation of the wearable device.

2. . The wearable device according to claim 1, wherein the wearable device is a spectacle frame.

3. . The wearable device according to any of claim 1 or 2, wherein the heart rate sensing module is configured so that when said wearable device is worn by the user the distance between the contactless sensor (4) and the skin of the user is greater than or equal to 0.1 mm and smaller than or equal to 5 mm.

4. . The wearable device according to any of the preceding claims, wherein when the wearable device is worn by the user, the contactless sensor (4) faces an artery or vein of the user.

5. . The wearable device according to any of the preceding claims, wherein the contactless sensor (4) is a capacitive sensor.

6. . The wearable device according to the preceding claim, wherein the at least one parameter relates to the capacitance measured by the capacitive sensor.

7. . The wearable device according to any of claims 1 to 4, wherein the contactless sensor (4) is a magnetic sensor.

8. . The wearable device according to any of the preceding claims, wherein the computer executable instructions of the heart rate determining component further comprises instructions for determining heart rate data based on the variation over time of the distance between the at least a reference point of the skin of the user and a reference point of the module.

9. . The wearable device according to any of the preceding claims, wherein the heart determining component further comprises an information generating unit configured to generate information based on the heart rate data.

10. . The wearable device according to the preceding claim, wherein the information comprises recommendation data including a change of activity recommendation and/or an alert indicative of the user's heart state and/or an access to a service offer.

11. . The wearable device according to any of the preceding claims, wherein the communication unit (12) is configured to send heart rate related data to a distant entity, for example a wearable computer device and/or a personal computer device, associated to the user of the heart rate determining component.

**Patentansprüche**

1. Tragbare ("Wearable") Vorrichtung, umfassend eine Herzfrequenzbestimmungskomponente (10) zum Bestimmen der Herzfrequenz eines Trägers der Wearable-Vorrichtung und ein Herzfrequenzabfühlmodul (2), das auf der Wearable-Vorrichtung fixiert ist, wobei die Wearable-Vorrichtung vorgesehen ist, um von einem Benutzer getragen zu werden, wobei das Modul umfasst:

mindestens einen kontaktlosen Sensor (4), der vorgesehen ist, um mindestens einen Parameter abzufühlen, der den Abstand zwischen mindestens einem Referenzpunkt der Haut des Benutzers und einem Referenzpunkt des Moduls angibt, wenn die Wearable-Vorrichtung von dem Benutzer in einer solchen Weise getragen wird, dass der kontaktlose Sensor in einem Abstand zu der Haut des Benutzers ist,
mindestens eine Kommunikationsentität (6), die vorgesehen ist, um Daten zu kommunizieren, die der Herzfrequenzbestimmungskomponente den mindestens einen Parameter angeben, der durch den mindestens einen kontaktlosen Sensor abgefühlt wurde,
wobei die Herzfrequenzbestimmungskomponente umfasst:

eine Kommunikationseinheit (12), die ausgestaltet ist, um Daten zu empfangen, die den mindestens einen Parameter angeben, der von dem mindestens einen kontaktlosen Sensor (4) des Herzfrequenzabfühlmoduls abgefühlt wurde, und
eine Herzfrequenzeinheit (14), umfassend mindestens
einen Speicher (141), der ausgestaltet ist, um computerausführbare Anweisungen zu speichern; und

einen Prozessor (142) zum Ausführen der computerausführbaren Anweisungen, wobei die computerausführbaren Anweisungen Anweisungen zum Bestimmen von Herzfrequenzdaten umfassen, die die Herzfrequenz des Benutzers basierend auf den empfangenen Daten angeben, **dadurch gekennzeichnet, dass** die Wearable-Vorrichtung des Weiteren umfasst:

einen von einem Laufzeitsensor, der zum Abfühlen des Abstands zwischen der Wearable-Vorrichtung und dem Benutzer der Wearable-Vorrichtung ausgestaltet ist, und einem Näherungssensor auf Infrarotreflexionsbasis, um die Nähe zwischen der Wearable-Vorrichtung und dem Benutzer der Wearable-Vorrichtung abzufühlen, und wobei die Wearable-Vorrichtung des Weiteren umfasst: einen Beschleunigungsmesser und/oder ein Gyroskop, der/das ausgestaltet ist/sind, um die Orientierung der Wearable-Vorrichtung abzufühlen.

2. Wearable-Vorrichtung nach Anspruch 1, wobei die Wearable-Vorrichtung ein Brillengestell ist.

3. Wearable-Vorrichtung nach einem von Anspruch 1 oder 2, wobei das Herzfrequenzabfühlmodul so ausgestaltet ist, dass, wenn die Wearable-Vorrichtung von dem Benutzer getragen wird, der Abstand zwischen dem kontaktlosen Sensor (4) und der Haut des Benutzers größer als oder gleich 0,1 mm und kleiner gleich oder gleich 5 mm ist.

4. Wearable-Vorrichtung nach einem der vorhergehenden Ansprüche, wobei der kontaktlose Sensor (4), wenn die Wearable-Vorrichtung von dem Benutzer getragen wird, zu einer Arterie oder Vene des Benutzers weist.

5. Wearable-Vorrichtung nach einem der vorhergehenden Ansprüche, wobei der kontaktlose Sensor (4) ein kapazitiver Sensor ist.

6. Wearable-Vorrichtung nach einem der vorhergehenden Ansprüche, wobei der mindestens eine Parameter die durch den kapazitiven Sensor gemessene Kapazität betrifft.

7. Wearable-Vorrichtung nach einem der Ansprüche 1 bis 4, wobei der kontaktlose Sensor (4) ein magnetischer Sensor ist.

8. Wearable-Vorrichtung nach einem der vorhergehenden Ansprüche, wobei die computerausführbaren Anweisungen der Herzfrequenzbestimmungskomponente des Weiteren Anweisungen zum Bestimmen von Herzfrequenzdaten basierend auf der zeitlichen Variation des Abstands zwischen dem mindestens einen Referenzpunkt der Haut des Benutzers und einem Referenzpunkt des Moduls umfassen.

9. Wearable-Vorrichtung nach einem der vorhergehenden Ansprüche, wobei die Herzbestimmungskomponente des Weiteren eine Informationsgenerierungseinheit umfasst, die ausgestaltet ist, um Informationen basierend auf den Herzfrequenzdaten zu generieren.

10. Wearable-Vorrichtung nach dem vorhergehenden Anspruch, wobei die Informationen Empfehlungsdaten umfassen, die eine Empfehlung zur Änderung der Aktivität und/oder einen Alarm, der den Herzstatus des Benutzers angibt, und/oder einen Zugang zu einem Serviceangebot einschließen.

11. Wearable-Vorrichtung nach einem der vorhergehenden Ansprüche, wobei die Kommunikationseinheit (12) ausgestaltet ist, um mit der Herzfrequenz zusammenhängende Daten an eine entfernt befindliche Entität zu senden, beispielsweise eine Wearable-Computervorrichtung und/oder eine Personal Computer (PC)-Vorrichtung, die mit dem Benutzer der Herzfrequenzbestimmungskomponente assoziiert ist.

**Revendications**

1. Dispositif portable comprenant un composant de détermination de la fréquence cardiaque (10) pour déterminer la fréquence cardiaque d'un porteur du dispositif portable et un module de détection de la fréquence cardiaque (2) fixé sur le dispositif portable, le dispositif portable étant destiné à être porté par un utilisateur, le module comprenant :

au moins un capteur sans contact (4) adapté pour détecter au moins un paramètre indiquant la distance entre au moins un point de référence de la peau de l'utilisateur et un point de référence du module lorsque le dispositif portable est porté par l'utilisateur de telle sorte que le capteur sans contact est à distance de la peau de l'utilisateur, au moins une entité de communication (6) adaptée pour communiquer des données indiquant l'au moins un paramètre détecté par l'au moins un capteur sans contact au composant de détermination de la fréquence cardiaque, le composant de détermination de la fréquence cardiaque comprenant :

une unité de communication (12) configurée pour recevoir des données indiquant au moins un paramètre détecté par l'au moins un capteur sans contact (4) du module de détection de la fréquence cardiaque, et une unité de fréquence cardiaque (14) comprenant au moins :

une mémoire (141) configurée pour stocker des instructions exécutables par ordinateur ; et
un processeur (142) pour exécuter les instructions exécutables par ordinateur, dans lequel les instructions exécutables par ordinateur comprennent des instructions pour déterminer des données de fréquence cardiaque indiquant la fréquence cardiaque de l'utilisateur sur la base des données reçues, **caractérisé en ce que** le dispositif portable comprend en outre :

l'un d'un capteur de temps de vol configuré pour détecter la distance entre le dispositif portable et l'utilisateur du dispositif portable et d'un capteur de proximité basé sur la réflectance infrarouge pour détecter la proximité entre le dispositif portable et l'utilisateur du dispositif portable,
et **en ce que** le dispositif portable comprend en outre :
un accéléromètre et/ou un gyroscope configurés pour détecter l'orientation du dispositif portable.

2. Dispositif portable selon la revendication 1, dans lequel le dispositif portable est une monture de lunettes.

3. Dispositif portable selon l'une quelconque des revendications 1 ou 2, dans lequel le module de détection de la fréquence cardiaque est configuré de sorte que, lorsque le dispositif portable est porté par l'utilisateur, la distance entre le capteur sans contact (4) et la peau de l'utilisateur est supérieure ou égale à 0,1 mm et inférieure ou égale à 5 mm.

4. Dispositif portable selon l'une quelconque des revendications précédentes, dans lequel lorsque le dispositif portable est porté par l'utilisateur, le capteur sans contact (4) est en regard d'une artère ou d'une veine de l'utilisateur

5. Dispositif portable selon l'une quelconque des revendications précédentes, dans lequel le capteur sans contact (4) est un capteur capacitif.

6. Dispositif portable selon la revendication précédente, dans lequel l'au moins un paramètre se rapporte à la capacité mesurée par le capteur capacitif.

7. Dispositif portable selon l'une quelconque des revendications 1 à 4, dans lequel le capteur sans contact (4) est un capteur magnétique.

8. Dispositif portable selon l'une quelconque des revendications précédentes, dans lequel les instructions exécutables par ordinateur du composant de détermination de la fréquence cardiaque comprennent en outre des instructions pour déterminer des données de fréquence cardiaque sur la base de la variation dans le temps de la distance entre l'au moins un point de référence de la peau de l'utilisateur et un point de référence du module.

9. Dispositif portable selon l'une quelconque des revendications précédentes, dans lequel le composant de détermination du coeur comprend en outre une unité de génération d'informations configurée pour générer des informations sur la base des données de fréquence cardiaque.

10. Dispositif portable selon la revendication précédente, dans lequel les informations comprennent des données de recommandation, comprenant une recommandation de changement d'activité et/ou une alerte indiquant l'état du coeur de l'utilisateur et/ou un accès à une offre de service.

11. Dispositif portable selon l'une quelconque des revendications précédentes, dans lequel l'unité de communication (12) est configurée pour envoyer des données relatives à la fréquence cardiaque à une entité distante, par exemple un dispositif informatique portable et/ou un dispositif informatique personnel, associé à l'utilisateur du composant de détermination de la fréquence cardiaque.

Fig. 1

Fig. 2

Fig. 3

COM

PROC

MEM

20

Fig. 4

50

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015051468 A1
- US 2015045650 A1
- WO 2010140106 A1
- US 2014200469 A1